Europäisches Patentamt

European Patent Office

Office européen des brevets

(11) Veröffentlichungsnummer: **0 467 218 A2**

## EUROPÄISCHE PATENTANMELDUNG

(21) Anmeldenummer: **91111536.8**

(22) Anmeldetag: **11.07.91**

(51) Int. Cl.⁵: **A61K 7/48**

(30) Priorität: **17.07.90 DE 4022644**

(43) Veröffentlichungstag der Anmeldung:
**22.01.92 Patentblatt 92/04**

(84) Benannte Vertragsstaaten:
**AT BE CH DE ES FR GB IT LI NL**

(71) Anmelder: **Beiersdorf Aktiengesellschaft**
**Unnastrasse 48**
**W-2000 Hamburg 20(DE)**

(72) Erfinder: **Schönrock, Uwe, Dr.**
**Mittelstrasse 18b**
**W-2000 Norderstedt(DE)**
Erfinder: **Stäb, Franz, Dr.**
**Kalveslohtwiete 13**
**W-2000 Hamburg 13(DE)**
Erfinder: **Ebens, Bodo**
**Alma-Rogge-Strasse 13**
**W-2820 Bremen(DE)**
Erfinder: **Brinkmann, Silke**
**Damm 96**
**W-2080 Pinneberg(DE)**
Erfinder: **Christiansen, Michael**
**Pinnausring 63**
**W-2082 Tornesch(DE)**
Erfinder: **Kröpke, Rainer**
**Glockenweg 6**
**W-2095 Marschacht(DE)**
Erfinder: **Thormählen, Sven, Dr.**
**38, Glen Street**
**Greenwich, CT 06831(US)**
Erfinder: **Alert, Dirk, Dr.**
**Beselerstrasse 8**
**W-2000 Hamburg 52(DE)**
Erfinder: **Sauermann, Gerhard, Dr.**
**Hambrook 14**
**W-2351 Wiemersdorf(DE)**

(54) **Hautpflegende Wirkstoffkombinationen.**

㊄ Lipidkombinationen für kosmetische Zwecke, enthaltend

```
0,0 - 95,0 % ungesättigte Fettsäuren und/oder deren
             Tocopherylester
0,0 - 65,0 % n-Alkane
0,0 - 30,0 % Squalen
0,0 - 50,0 % Cholesterin und/oder Wollwachsalkohol
0,0 - 80,0 % Triglyceride
0,0 - 60,0 % Wachsester,
```

mit der Maßgabe, daß wenigstens zwei der vorgenannten Substanzklassen in den Lipidkombination enthalten sind.

Die vorliegende Erfindung betrifft kosmetische Wirkstoffkombinationen, die zur Hautpflege geeignet sind.

Unter Hautpflege im Sinne der Erfindung ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, Elektrolyten) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Die Haut besteht aus zwei Hauptkomponenten: aus verhornenden, proteinreichen Keratinozyten und aus dem mit Lipiden angereicherten Raum zwischen den einzelnen Zellen. Die Struktur der Haut ist also einer Backsteinmauer vergleichbar. Die Zellen stellen dabei die Steine dar und die Lipide den Mörtel.

Die multilamellar angeordneten Lipide spielen beim Aufbau der Permeabilitätsbarriere der Haut eine wichtige Rolle; es ist vorwiegend ihre chemische Zusammensetzung welche die selektive Permeabilität der Haut bewirkt.

Die interzellularen Lipide haben etwa folgende Zusammensetzung (P.M. Elias, "Structure and Function of the Stratum Corneum Permeability Barrier", Drug Development Research 13, 97 - 105, 1988):

| | | |
|---|---|---|
| 1) | Polare Lipide | 5 % |
| 2) | Cholesterylsulfat | 2 % |
| 3) | Neutrallipide | 78 % |
| | davon | |
| | - freie Sterole | 14 % |
| | - freie Fettsäuren | 19 % |
| | - Triglyceride | 25 % |
| | - Sterol/Wachsester | 5 % |
| | - Squalen | 5 % |
| | - n-Alkane | 6 % |
| 4) | Sphingolipide | 18 % |
| | davon | |
| | - Glucosylceramide | Spuren |
| | - Ceramide | ca. 18 % |

Bei der Reinigung der Haut sollen Schmutz, Talg, Schweiß und Schuppen von der Haut entfernt werden. Da der Schmutz teilweise von abschilfernden Epithelien und körpereigenen Lipiden umgeben ist, ist die Hautreinigung ohne Beeinträchtigung der Hornschicht nicht möglich. Durch waschaktive Substanzen (Seifen, Detergentien usw.), welche gerade die lipophilen Hautbestandteile entfernen, wird diese Schädigung noch verstärkt.

Herkömmliche Hautreinigung ist also deutlich zu unterscheiden von Hautpflege und von pflegender Hautreinigung im Sinne der vorliegenden Erfindung.

Ziel der Hautpflege ist es, den durch tägliche Waschen verursachten Fett- und Wasserverlust der Haut auszugleichen. Dies ist gerade dann wichtig, wenn das natürliche Regenrationsvermögen nicht ausreicht. Außerdem soll sie vor Umwelteinflüssen, insbesondere vor Sonne und Wind, schützen und die Hautalterung verzögern.

Folgende Methoden der täglichen Hautpflege sind an sich bekannt:

a) Okklusion der Haut

Wird die Haut mit einem Lipidfilm (herkömmliche Salben oder Crèmes) bedeckt, wird die Barrierefunktion der Haut nicht wiederhergestellt. Der Lipidfilm stellt nur eine physikalische Barriere dar, die zwar verhindert, daß die Haut austrocknet.

Mit Reinigungsmitteln kann dieser Film aber leicht abgewaschen werden, so daß wieder der ursprüngliche, beeinträchtigte Zustand erreicht ist.

Auf die Hautregeneration hat diese Methode an sich keinen Einfluß. Bestenfalls wird der Status quo der Haut erhalten.

b) Behandlung der Haut mit essentiellen Fettsäuren

Essentielle Fettsäuren werden derzeit gelegentlich in dermatologischen Präparaten zur Behandlung von trockener Haut eingesetzt. Eindeutige Beweise zu deren Wirksamkeit stehen aber noch aus.

c) Behandlung der Haut mit keratolytisch wirksamen Substanzen (z.B. Harnstoff, Salicylsäure, Hydroxycarbonsäuren usw.). Diese Substanzen wirken je nach verwendeter Konzentration keratolytisch, proteolytisch, wasserbindend, penetrationsfördernd, epidermisverdünnend oder juckreizstillend. Ihre Verwendung ist im wesentlichen auf medizinische Indikationen beschränkt.

d) Behandlung mit feuchtigkeitsregulierenden Substanzen (z.B. Glycerin, Sorbit usw.) Diese Stoffe sind hygroskopischer Natur und bewirken in höheren Konzentrationen, daß die Haut in verstärktem Maße zum Austrocknen neigt.

Aufgabe der vorliegenden Erfindung war es also, die Nachteile des Standes der Technik zu beseitigen. Insbesondere sollten hautpflegende Wirkstoffkombinationen zur Verfügung gestellt werden, welche den durch die tägliche Reinigung der Haut verursachten Fett- und Wasserverlust der Haut ausgleichen, zumal dann, wenn die natürliche Regeneration nicht ausreicht. Weiterhin soll die Haut vor Umwelteinflüssen wie Sonne und Wind geschützt werden.

Es hat sich überraschenderweise gezeigt, und darin liegt die Lösung der Aufgabe, daß Lipidkombinationen für kosmetische Zwecke, enthaltend

```
0,0 - 95,0 % ungesättigte Fettsäuren und/oder deren
             Tocopherylester
0,0 - 65,0 % n-Alkane
0,0 - 30,0 % Squalen
0,0 - 50,0 % Cholesterin und/oder Wollwachsalkohol
0,0 - 80,0 % Triglyceride
0,0 - 60,0 % Wachsester,
```

mit der Maßgabe, daß wenigstens zwei der vorgenannten Substanzklassen in den Lipidkombinationen enthalten sind, den Nachteilen des Standes der Technik abhelfen.

Vorteilhaft enthalten die erfindungsgemäßen Lipidkombinationen

```
5,0 - 95,0 % ungesättigte Fettsäuren und/oder
             deren Tocopherylester
95,0 -  5,0 % Cholesterin und/oder Wollwachsalkohol
```

Als günstig haben sich aber auch Lipidkombinationen erwiesen, enthaltend

```
7,0 - 60,0 % ungesättigte Fettsäuren und/oder deren
             Tocopherylester
3,0 - 15,0 % n-Alkane
0,1 -  6,5 % Squalen
0,5 - 20,0 % Cholesterin und/oder Wollwachsalkohol
10,0 - 40,0 % Triglyceride
5,0 - 25,0 % Wachsester.
```

Bevorzugt enthalten die erfindungsgemäßen Lipidkombinationen

```
10,0 - 40,0 % ungesättigte Fettsäuren und/oder deren
               Tocopherylester
 5,0 - 12,0 % n-Alkane
 0,1 -  6,5 % Squalen
 1,0 - 18,0 % Cholesterin und/oder Wollwachsalkohol
18,0 - 35,0 % Triglyceride
15,0 - 22,0 % Wachsester.
```

Ganz besonders bevorzugt enthalten die erfindungsgemäßen Lipidkombinationen

```
15,0 - 38,0 % ungesättigte Fettsäuren und/oder deren
               Tocopherylester
 7,0 - 10,0 % n-Alkane
 0,1 -  6,5 % Squalen
 1,0 - 10,0 % Cholesterin und/oder Wollwachsalkohol
18,0 - 35,0 % Triglyceride
15,0 - 22,0 % Wachsester.
```

Im Rahmen dieser Anmeldung sind stets Gewichtsprozente, bezogen auf 100 % der Gesamtzusammensetzung der jeweiligen erfindungsgemäßen Kombination gemeint.

Die ungesättigten Fettsäuren werden bevorzugt gewählt aus der Gruppe der linearen und verzweigten Fettsäuren mit 8 - 25 Kohlenstoffatomen. Besonders bevorzugt sind Linolsäure, Linolensäure, Ölsäure bzw. deren Tocopherylester, insbesondere Vitamin E-Linoleat und Vitamin E-Oleat. Dabei spielt es keine grundlegende Rolle, welcher Tocopheryl-Grundkörper gewählt wird.

Die n-Alkane werden bevorzugt gewählt aus der Gruppe der Squalane.

Unter "Triglyceriden" im Sinne dieser Patentanmeldung sind Verbindungen zu verstehen, in denen Glycerin mit drei organischen Säureresten verestert ist.

Die Triglyceride werden bevorzugt gewählt aus der Gruppe der Glycerylcaproate, -caprylate, -linoleate, -stearate, -palmitate.

Insbesondere sind vorteilhaft die Triglyceride gewählt aus der Gruppe
- Glyceryldicaproatmonocaprylat, Glyceryltricaproat, bzw. beliebige Gemische daraus (z.B. Miglyol 812 von Dynamit Nobel),
- Glyceryldicaproatmonolinoleat (vorteilhaft in beliebigen Gemischen mit Glyceryldicaproatmonocaprylat und/oder Glyceryltricaproat (z.B. Miglyol 818 von Dynamit Nobel),
- Glycerylmonostearatdipalmitat, Glyceryldistearatmonopalmitat, bzw. beliebige Gemische daraus (z.B. Emulgator GMS von Th.Goldschmidt).

Das Cholesterin und/oder die Wollwachsalkohole können aus dem gereinigten Wollfett des Schafes erhalten werden. Als Wollwachsalkohole können Handelsprodukte wie z.B. Eucerinum Anhydricum[R], Beiersdorf Aktiengesellschaft, verwendet werden.

Als Wachsester hat sich das Jojobaöl als besonders vorteilhaft herausgestellt.

Die Squalene können vorteilhaft aus den aus Fischleberöl, insbesondere Haifischleberöl gewonnenen Produkten gewählt werden.

Es hat sich als besonders vorteilhaft erwiesen, das Cholesterin in Lösungsmitteln, besonders bevorzugt ist Cetearyloctanoat, zu lösen und den Formulierungen als Lösung zuzugeben.

Die erfindungsgemäßen Lipidkombinationen können vorteilhaft als solche eingesetzt werden, es ist aber auch möglich und vorteilhaft, diese Kombinationen in übliche kosmetische Grundlagen einzuarbeiten. Dabei sind die üblichen Regeln der kosmetischen Fertigungspraxis zu beachten, welche dem Fachmanne geläufig sind. Ferner weiß der Fachmann, daß solchen Produkten übliche Hilfs- und/oder Zusatzstoffe zugefügt werden können, ohne daß der Rahmen der vorliegenden Erfindung dadurch verlassen würde.

Hilfs- und Zusatzstoffe sind beispielsweise konsistenzgebende Mittel Füllstoffe, Parfum, Farbstoffe, Emulgatoren, zusätzliche Wirkstoffe wie Vitamine oder Proteine, Lichtschutzmittel, Stabilisatoren, Antioxidantien, Konservierungsmittel, Insektenrepellentien, Alkohol, Wasser, Salze, proteolytisch oder keratolytisch wirksame Substanzen usw.

Die folgenden Beispiele sind dazu gedacht, den Gegenstand der vorliegenden Erfindung zu erläutern, nicht etwa, ihn auf diese Beispiele zu beschränken.

| Beispiel | I | II | III | IV |
|---|---|---|---|---|
| alpha-Tocopheryllinoleat | 18,0 | 36,0 | 18,0 | 18,0 |
| Glyceryl- di/tri-caproat/ -caprylat/-linoleat | 34,2 | 20,2 | 22,0 | 34,2 |
| Cholesterin | 1,5 | 1,5 | 8,5 | - |
| Squalan | 10,0 | 8,0 | 10,0 | 10,0 |
| Squalen | 0,5 | 0,5 | 1,0 | - |
| Jojobaöl | 16,0 | 16,0 | 20,0 | 20,0 |
| Antioxidant | 0,8 | 0,8 | 1,5 | 0,8 |
| Cetaryloctanoat | 19,0 | 17,0 | 19,0 | 17,0 |
| | 100,0 | 100,0 | 100,0 | 100,0 |

Die Zusammensetzungen gemäß den Beispielen I - IV können entweder als solche für kosmetische Zwecke verwendet werden oder den üblichen kosmetischen Grundlagen zugefügt werden.

Wenn die Zusammensetzungen in kosmetische Grundlagen eingearbeitet werden sollen, hat es sich als günstig erwiesen, 0,1 - 35,0 Teile der erfindungsgemäßen Zusammensetzungen mit 99,9 - 65,0 Teilen der jeweilig geeigneten kosmetischen Grundlage zu einer hautpflegenden Formulierung zu vereinen.

Dabei bestehen keine Beschränkungen in Bezug auf das gewählte Kosmetikum. Die erfindungsgemäßen Zusammensetzungen lassen sich allen kosmetischen Grundlagen zufügen. Grundsätzlich sind allerdings nichtwäßrige Zusammensetzungen, W/O- und O/W-Emulsionen bevorzugt.

Der Fachmann weiß dabei, welche Gesichtspunkte bei der Zusammenstellung der Formulierungen zu beachten sind.

Vorteilhaft können die erfindungsgemäßen Lipidkombinationen in Pflegeprodukten wie beispielsweise O/W-Crèmes, W/D-Crèmes, O/W-Lotionen, W/O-Lotionen, Hautölen, Reinigungsmitteln, Shampoos usw. eingesetzt werden.

In den Beispielen V - X sind vorteilhafte kosmetische Formulierungen, enthaltend erfindungsgemäße Lipidkombinationen, aufgeführt.

Beispiel V

O/W-Tagescrème

**Fettphase:**

Octyldodecanol
(Emulgade F, Henkel KGaA)                    100,00
Cetearylalkohol/TEG-40-Castor Oil/
Natriumcetearylsulfat
(Eutanol G, Henkel KGaA)                     40,00
Paraffinöl
(Mineralöl 5 E, Shell)                       15,00

| Wasserphase: | |
|---|---|
| Glycerin DAB 9 | 50,00 |
| Wasser VES | 787,00 |

In die 75°C warme Fettphase wird ein Gemisch aus 3,00 Teilen der Lipidkombination gemäß Beispiel II und 5,00 Teilen Paraffinöl eingerührt und mit der Wasserphase bei einer Temperatur von 75°C verrührt und homogenisiert, bis eine gleichmäßige, weiße Crème entstanden ist.

Beispiel VI

Hautcrème vom W/O-Typ

**Fettphase:**

Dicocodistearylpentaerythritylcitrat
(Dehymuls F, Henkel KGaA)                    80,00
Myristylmyristat
(Cetiol MM, Henkel KGaA)                     50,00
Cetearylalkohol/TEG-40-Castor Oil/
Natriumcetearylsulfat
(Eutanol G, Henkel KGaA)                     30,00
Vaseline DAB 9                               150,00
Paraffinöl
(Mineralöl 5 E, Shell)                       23,00

| Wasserphase | |
|---|---|
| Glycerin DAB 9 | 50,00 |
| $MgSO_4$ | 3,00 |
| Wasser VES | 607,00 |

In die 75°C warme Fettphase wird ein Gemisch aus 2,00 Teilen der Lipidkombination gemäß Beispiel II und 5,00 Teilen Paraffinöl gelöst. Die Fettphase wird sodann zu der 75°C warmen Wasserphase gegeben, verrührt und homogenisiert, bis eine gleichmäßige weiße Crème entstanden ist.

Beispiel VII

O/W-Lotion

```
Fettphase:


Octyldodecanol
(Emulgade F, Henkel KGaA)              60,00
Cetearylalkohol/TEG-40-Castor Oil/
Natriumcetearylsulfat
(Eutanol G, Henkel KGaA)               95,00
Cetearylisononanoat
(Cetiol SN, Henkel KGaA)               80,00
```

| Wasserphase: | |
|---|---|
| Glycerin DAB 9 | 50,00 |
| Wasser VES | 707,00 |

In die 75°C warme Fettphase wird ein Gemisch aus 3,00 Teilen der Lipidkombination gemäß Beispiel I und 5,00 Teilen Eutanol G gegeben. Die Fettphase wird sodann zur 75°C warmen Wasserphase gegeben, bei 60°C verrührt und bei 30°C homogenisiert, bis eine gleichmäßige Lotion entstanden ist.

Beispiel VIII

Antitranspirantcrème vom O/W-Typ

Fettphase:

| | |
|---|---:|
| Cetearylalkohol | |
| (Lanette O, Henkel KGaA) | 80,00 |
| Ceteareth-12 | |
| (Eumulgin B1, Henkel KGaA) | 15,00 |
| Ceteareth-20 | |
| (Eumulgin B2, Henkel KGaA) | 15,00 |
| Cetearylalkohol/TEG-40-Castor Oil/ | |
| Natriumcetearylsulfat | |
| (Eutanol G, Henkel KGaA) | 75,00 |

Wasserphase:

| | |
|---|---:|
| Aluminiumhydroxychlorid | |
| (Locron S, Hoechst) | 200,00 |
| Wasser VES | 608,00 |

In die 75°C warme Fettphase wird ein Gemisch aus 2,00 Teilen der Lipidkombination gemäß Beispiel IV gegeben. Die Fettphase wird sodann zur 75°C warmen Wasserphase gegeben, verrührt und bei 30°C homogenisiert, bis eine gleichmäßige, weiße Crème entstanden ist.

Beispiel IX

Shampoo:

| | |
|---|---:|
| Natriumlaurethsulfat | |
| (Texapon NSO, Henkel KGaA) | 150,00 |
| Cocoamidopropylbetain | |
| (Tegobetain L7, Th.Goldschmidt) | 25,00 |
| Cocamid DEA | |
| Komperlan COD, Henkel KGaA) | 10,00 |
| NaCl | 10,00 |
| Lipidkombination gemäß | |
| Beispiel I | 0,50 |
| Wasser VES | ad 500,00 |

Die Komponenten werden bei 25°C verrührt, bis eine gleichmäßige, klare Mischung entstanden ist.

Beispiel X

Hautöl:

| | |
|---|---|
| Caprylic/Caprictriglyceride | |
| (Miglyol 812, Dynamit Nobel) | 100,00 |
| Hexyllaurat | |
| (Cetiol A, Henkel KGaA) | 100,00 |
| Octylstearat | |
| (Cetiol 886, Henkel KGaA) | 100,00 |
| Paraffinöl | |
| (Mineralöl 5 E, Shell) | 190,00 |
| Lipidkombination gemäß | |
| Beispiel II | 10,00 |

Die Komponenten werden bei 25° C verrührt, bis eine gleichmäßige, klare Mischung entstanden ist.

Die Formulierungen entsprechend den Beispielen I - X sind hochwertige kosmetische Produkte. Nach regelmäßiger Anwendung ist die Haut im Vergleich zu herkömmlichen Formulierungen (etwa normalen W/O- oder O/W-Crèmes) geglättet.

Die Reinigungsprodukte gemäß der vorliegenden Erfindung zeichnen sich gleichermaßen durch erhöhte hautpflegende Wirkung gegenüber den herkömmlichen Formulierungen aus, ohne aber Reinigungskraft einzubüßen.

**Patentansprüche**

1. Lipidkombinationen für kosmetische Zwecke, enthaltend

    0,0 - 95,0 % ungesättigte Fettsäuren und/oder deren
    Tocopherylester
    0,0 - 65,0 % n-Alkane
    0,0 - 30,0 % Squalen
    0,0 - 50,0 % Cholesterin und/oder Wollwachsalkohol
    0,0 - 80,0 % Triglyceride
    0,0 - 60,0 % Wachsester,

    mit der Maßgabe, daß wenigstens zwei der vorgenannten Substanzklassen in den Lipidkombination enthalten sind.

2. Lipidkombinationen für kosmetische Zwecke, enthaltend

    5,0 - 95,0 % ungesättigte Fettsäuren und/oder
    deren Tocopherylester
    95,0 -  5,0 % Cholesterin und/oder Wollwachsalkohol.

3. Lipidkombinationen nach Anspruch 1, enthaltend

```
 7,0 - 60,0 % ungesättigte Fettsäuren und/oder deren
               Tocopherylester
 3,0 - 15,0 % n-Alkane
 0,1 -  6,5 % Squalen
 0,5 - 20,0 % Cholesterin und/oder Wollwachsalkohol
10,0 - 40,0 % Triglyceride
 5,0 - 25,0 % Wachsester.
```

4. Lipidkombinationen nach Anspruch 1, enthaltend

```
10,0 - 40,0 % ungesättigte Fettsäuren und/oder deren
               Tocopherylester
 5,0 - 12,0 % n-Alkane
 0,1 -  6,5 % Squalen
 1,0 - 18,0 % Cholesterin und/oder Wollwachsalkohol
18,0 - 35,0 % Triglyceride
15,0 - 22,0 % Wachsester.
```

5. Lipidkombinationen nach Anspruch 1, enthaltend

```
15,0 - 38,0 % ungesättigte Fettsäuren und/oder deren
               Tocopherylester
 7,0 - 10,0 % n-Alkane
 0,1 -  6,5 % Squalen
 1,0 - 10,0 % Cholesterin und/oder Wollwachsalkohol
18,0 - 35,0 % Triglyceride
15,0 - 22,0 % Wachsester.
```

6. Lipidkombinationen nach einem der Ansprüche 1 - 5,
dadurch gekennzeichnet, daß
die ungesättigten Fettsäuren gewählt werden aus der Gruppe der linearen und verzweigten Fettsäuren mit 8 - 25 Kohlenstoffatomen, bevorzugt Linolsäure, Linolensäure, Ölsäure bzw. deren Tocopherylester, insbesondere Vitamin E-Linoleat und Vitamin E-Oleat.

7. Lipidkombinationen nach einem der Ansprüche 1 - 6,
dadurch gekennzeichnet, daß
die n-Alkane gewählt werden aus der Gruppe Squalan und der Paraffine.

8. Lipidkombinationen nach einem der Ansprüche 1 - 7,
dadurch gekennzeichnet, daß
die Triglyceride gewählt werden aus der Gruppe
der Glycerylcaproate, -caprylate, -linoleate, -stearate, -palmitate, insbesondere die Triglyceride, gewählt aus der Gruppe
- Glyceryldicaproatmonocaprylat, Glyceryltricaproat, bzw. beliebige Gemische daraus,

- Glyceryldicaproatmonolinoleat (vorteilhaft in beliebigen Gemischen mit Glyceryldicaproatmonocaprylat und/oder Glyceryltricaproat),
- Glycerylmonostearatdipalmitat, Glyceryldistearatmonopalmitat, bzw. beliebige Gemische daraus.

9. Lipidkombinationen nach einem der Ansprüche 1 - 8, dadurch gekennzeichnet, daß die Wachsester als Jojobaöl gewählt werden.

10. Kosmetische Zusammensetzungen, enthaltend Lipidkombinationen gemäß einem der Ansprüche 1 - 9.